# FASCICULE DE BREVET EUROPEEN

(11) **EP 0 764 855 B1**
(45) Date de publication et mention de la délivrance du brevet: **05.09.2001**
(21) Numéro de dépôt: 96401428.6
(22) Date de dépôt: 27.06.1996
(51) Int. Cl.: G01T 1/29, G01T 1/164

(54) **Méthode d'obtention, en médecine nucléaire, d'une image du corps d'un patient corrigée des troncatures**
Verfahren zur Erzeugung eines Nuklearmedizin-Körperbildes mit Abstumpfungskorrektur
Method of obtaining, in nuclear medicine, an image of a patient corrected for truncation

(30) Priorité: 29.06.1995 FR 9507834
(43) Date de publication de la demande: 26.03.1997
(73) Titulaire: SMV INTERNATIONAL, 78534 Buc Cedex (FR)
(72) Inventeur: Guerard, Bruno, 92370 Chaville (FR); Papillon, Stéphane, 75013 Paris (FR)
(74) Mandataire: Schmit, Christian Norbert Marie

(56) Documents cités:
- EP-A- 0 200 939
- GB-A- 2 083 970
- US-A- 3 970 853
- KADRMAS,DJ ET AL.: "fb-tct truncation suppression by combining two shifted scans with extrapolation techniques" NUCLEAR SCIENCE SYMPOSIUM & MEDICAL IMAGING CONFERENCE,1994 IEEE CONFERENCE RECORD, 30 octobre 1994 - 15 novembre 1994, NORFOLK,VA.,USA, pages 1247-1250, XP002063019

## Description

L'invention concerne une méthode d'obtention, en médecine nucléaire, d'une image du corps d'un patient corrigée des troncatures.

La médecine nucléaire est une discipline dans laquelle on cherche à obtenir des informations d'un patient dans le corps duquel on a injecté une solution radioactive. Le principe de la méthode est le suivant. On injecte un marqueur radioémetteur gamma chez un patient. Ce marqueur, véhiculé par un agent biologique, se répand dans son corps et se fixe préférentiellement dans un ou plusieurs organes cibles. Une gamma caméra pourvue d'une tête détectrice capte le rayonnement gamma émis par le corps du patient pour un angle de vue donné. Ce rayonnement traverse un collimateur de la tête détectrice, excite un cristal scintillateur qui convertit l'énergie des photons gamma en une énergie lumineuse détectée par des tubes photo-multiplicateurs qui produisent alors, en fonction de l'intensité lumineuse reçue, des signaux électriques. En effectuant sur l'ensemble de ces signaux électriques des localisations barycentriques, on peut, d'une manière connue, déterminer l'origine de la scintillation dans un champ de détection. On réalise alors une acquisition incrémentale en cumulant le nombre de scintillations détectées sur tout le champ de détection de la tête détectrice et on acquiert ainsi, pour l'angle de vue précité, une image en projection ou projection révélatrice de la concentration du produit radioactif présente dans le corps du patient.

On a pris l'habitude d'acquérir une projection du corps du patient par angle de vue pour de multiples angles de vue régulièrement espacés sur un secteur angulaire donné. L'ensemble de ces projections permet de reconstruire, par un algorithme de calcul complexe, une image d'une section ou d'un volume du corps du patient.

Les projections précitées émises par le corps du patient présentent des artefacts dus à l'atténuation des rayonnements gamma traversant ledit corps. On corrige ces artefacts en obtenant une image en projection du corps du patient, acquise en transmission, à partir d'une source linéaire radioémettrice gamma que l'on dispose face à la tête détectrice, de manière à ce que le patient soit interposé entre ladite source et ladite tête détectrice. Une telle projection en transmission représente la transparence du patient aux rayonnements gamma dans un angle de vue donné.

Le champ de vue d'une gamma caméra est fonction des dimensions d'une surface utile de détection des détecteurs. Plus cette surface est étendue, plus le champ de vue de la gamma caméra est vaste. Toutefois, une surface de détection étendue grève le prix d'une gamma caméra et on réalise bien souvent des gamma caméras dont le champ de vue n'est pas suffisant pour permettre l'acquisition de projections englobant la totalité du corps du patient, ceci valant, en particulier, pour les patients corpulents.

De ce fait, les projections, acquises en émission ou en transmission, sont tronquées. Ces troncatures se manifestent par la présence d'artefacts sur l'image reconstruite du corps du patient dont les contours montrent une couronne de surbrillance qui rend l'ensemble de ladite image inexploitable dans l'optique d'une correction d'atténuation.

On connaît une méthode de correction des artefacts générés par les troncatures. Cette méthode propose, d'une part, une modélisation des contours du corps du patient et, d'autre part, une extrapolation des projections acquises par une fonction polynomiale décroissante jusqu'aux contours modélisés du corps du patient auxquels on affecte une valeur en atténuation égale à zéro.

Cependant, cette méthode peut conduire à l'obtention d'une image reconstruite corrigée de qualité plus médiocre qu'une image non corrigée. C'est le cas, en particulier, dans l'acquisition d'une image de correction d'atténuation en tomographie cardiaque. En effet, dans un tel cas, lorsqu'un poumon est tronqué, il n'est pas possible de restituer correctement la cage thoracique, l'extrapolation ayant lieu dans la cavité pulmonaire qui devrait être affectée d'une valeur en atténuation égale à zéro.

Le document EP-A-0 200 939 propose une méthode d'obtention d'une image du corps d'un patient conformément au préambule de la revendication 1.

La présente invention a pour but de proposer une nouvelle méthode d'obtention, en médecine nucléaire, d'une image du corps d'un patient, qui pallie à moindre coût les inconvénients précités et dans laquelle les troncatures sont corrigées sans extrapolation.

Ce but, ainsi que d'autres qui apparaîtront par la suite, est atteint grâce à une méthode d'acquisition selon laquelle on corrige une première projection tronquée par une seconde projection acquise dans une position voisine de la première projection. Aussi, dans un examen tomographique, on corrige une projection acquise dans une position α(i) tronquant une partie du corps du patient grâce à une combinaison des projections acquises dans des positions tomographiques α(i+1) et/ou α(i-1) voisines et ne tronquant pas la partie du corps du patient absente de la projection α(i).

L'invention a donc pour objet une méthode d'obtention, en médecine nucléaire, d'une image du corps d'un patient, telle que définie à la revendication 1.

La description qui va suivre, et qui ne comporte aucun caractère limitatif, permettra de mieux comprendre la manière dont l'invention peut être mise en pratique.

Elle doit être lue au regard des dessins annexés, dans lesquels :
- la figure 1 montre, en perspective, une gamma caméra pour la mise en oeuvre d'une méthode selon l'invention ;
- la figure 2 illustre, en coupe et de manière schématique, une tête détectrice d'une gamma caméra pour la mise en oeuvre d'une méthode selon l'invention ;
- la figure 3 schématise le principe d'une méthode selon l'invention ;
- la figure 4 représente le positionnement initial des têtes détectrices pour l'application d'une méthode selon l'invention ;
- la figure 5 représente les trajectoires empruntées par les têtes détectrices lors d'un examen tomographique conforme à une méthode selon l'invention ;
- la figure 6A schématise la projection d'un centre C lors d'une acquisition selon l'invention ; et
- la figure 6B schématise un centrage des images en projection acquises dans une méthode selon l'invention.

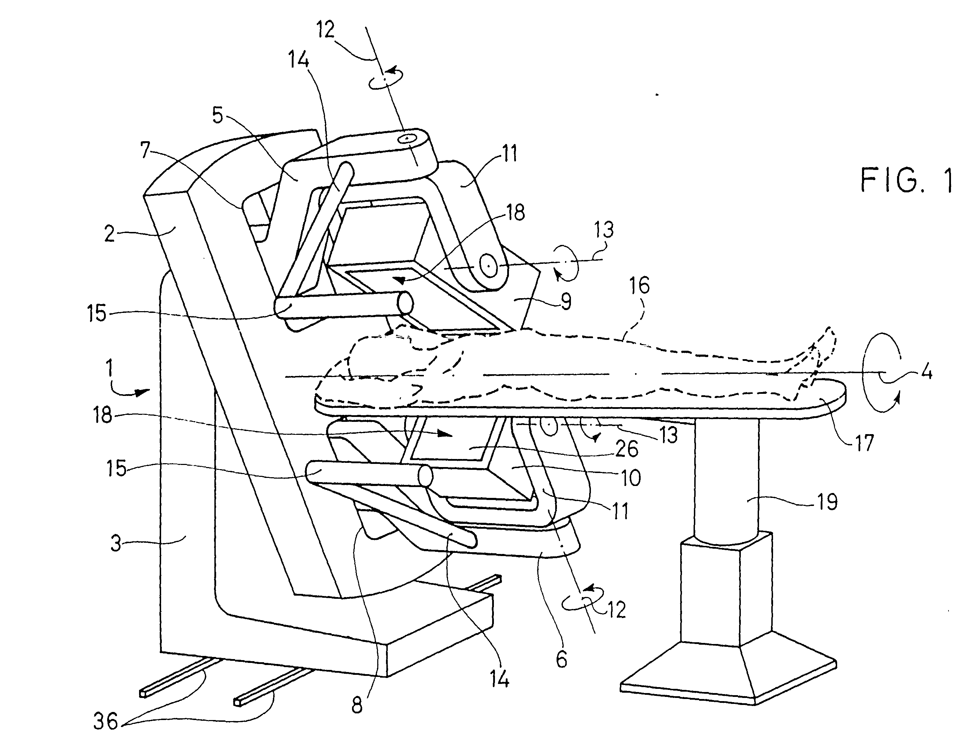

Une gamma caméra 1 pour la mise en oeuvre d'une méthode selon l'invention comporte, telle que représentée en figure 1, une embase 2 mobile sur un bâti 3 de manière à tourner en rotation autour d'un axe 4 appelé axe de rotation de la gamma caméra 1, ledit axe 4 étant sensiblement parallèle au sol et passant approximativement par le centre de gravité de ladite embase 2.

Le bâti 3 est mobile en translation latérale le long de rails 36 orthogonaux à l'axe 4.

L'embase 2 porte, dans l'exemple de réalisation de la présente description, deux bras 5 et 6 disposés de part et d'autre de l'axe 4, symétriquement. Toutefois, dans d'autres exemples de réalisation, l'embase 2 portera un unique bras voire plus de deux bras.

Les bras 5, 6 peuvent se rapprocher ou s'éloigner radialement de l'axe 4, dans les limites de fenêtres 7 et 8 de l'embase 2, grâce à un dispositif communément appelé ascenseur.

Chaque bras 5, 6 est muni, en son extrémité libre, d'une tête détectrice 9, 10, sensiblement parallélépipèdique et rectangle. L'accrochage de la tête détectrice 9, 10 au bras 5, 6 est réalisé par l'intermédiaire d'un étrier 11 en forme de U. L'étrier 11 est mobile en rotation par rapport à l'extrémité libre du bras 5 qui le porte selon un axe de rotation 12 orthogonal à l'axe 4 et appelé axe d'angulation de la tête détectrice 9, 10. En outre, la tête détectrice 9, 10 est mobile en rotation entre les bords descendants de l'étrier 11 selon un axe 13 parallèle à l'axe 4 et appelé axe d'orientation de la tête détectrice 9, 10.

D'autre part, chaque bras 5, 6 comporte un support 14 d'un boîtier 15 comportant une source linéaire radioémettrice gamma pour l'obtention de projections en transmission.

Un corps d'un patient 16 est placé allongé sur un lit 17, entre des faces actives 18 des têtes détectrices 9 et 10, sensiblement selon l'axe 4 de rotation de la gamma caméra 1. Le lit 17 est porté par un pied élévateur 19 qui permet de régler la hauteur du corps du patient 16 à une position voulue. Un tel lit 17 est décrit plus spécifiquement dans la demande de brevet française publiée sous le numéro 2 684 865 dont le contenu est ici incorporé par référence.

La gamma caméra 1, telle que précédemment décrite, autorise de nombreuses positions relatives des têtes détectrices 9, 10 par rapport au corps du patient 16. Cette richesse est une particularité des gamma caméras dites à statif ouvert, dans lesquelles les têtes détectrices 9, 10 ne sont pas enfermées dans un tunnel.

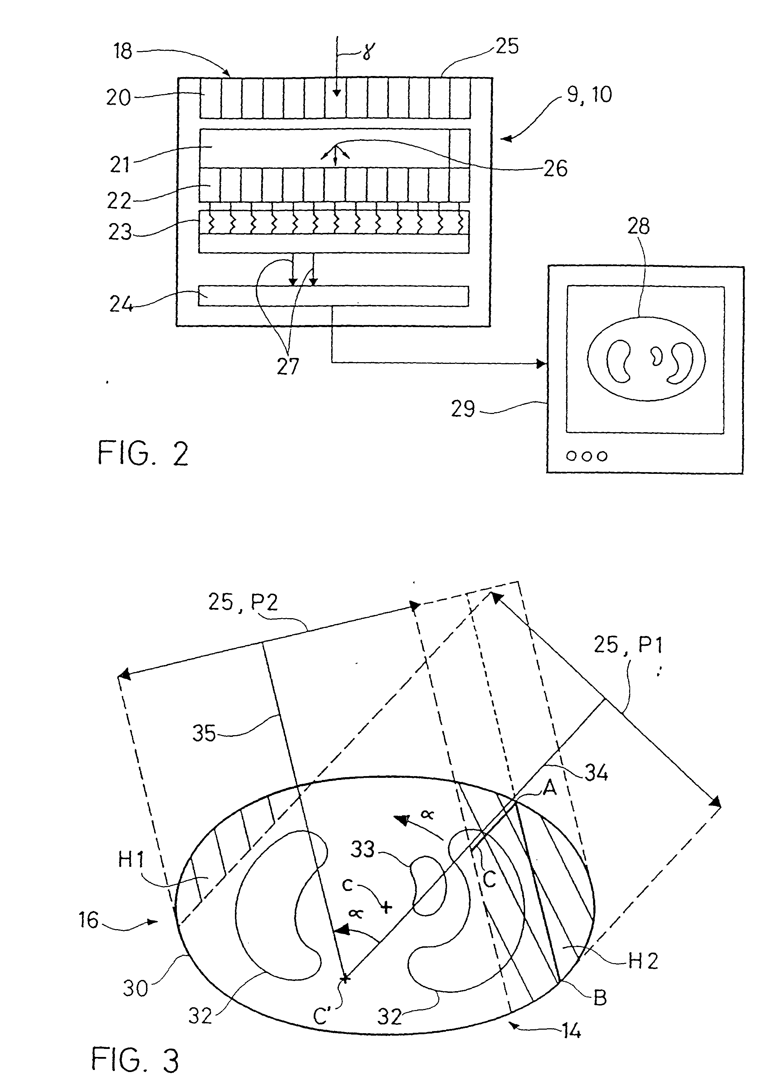

Ainsi que cela est représenté en figure 2, une tête détectrice 9, 10 comprend essentiellement, en partant de sa face active 18, un collimateur 20, un cristal scintillateur 21, un réseau de tubes photo-multiplicateurs 22, et des circuits de traitement 23 reliés à un circuit d'amplification 24.

Le collimateur 20 est généralement à trous droits. Il définit une surface de détection 25 de la tête détectrice 9, 10, c'est-à-dire une portion de la face active 18 de la tête détectrice 9, 10 susceptible de pouvoir détecter un rayonnement gamma γ.

Le principe d'obtention d'une image du corps d'un patient 16 est le suivant. Pour une position donnée en orientation et en angulation d'une tête détectrice 9, 10, les rayons gamma issus et/ou traversant le corps du patient 16, qui ont une direction de propagation sensiblement orthogonale à la surface de détection 25, traversent le collimateur 20 et provoquent une scintillation 26 dans cristal scintillateur 21. Cette scintillation 26 est détectée puis amplifiée par le réseau de tubes photo-multiplicateurs 22 qui élabore des signaux électriques de localisation 27. Ces signaux de localisation 27 sont ensuite traités et l'on obtient alors une image en projection ou projection du corps du patient 16 pour la position précitée. Avec plusieurs projections, obtenues pour des positions relatives différentes des têtes détectrices 9, 10 et du corps du patient 16, on peut reconstruire une image 28 du corps du patient 16, par exemple, une image en coupe dudit corps 16 qui, présentée sur un moniteur 29 relié à la gamma caméra 1, permet une analyse médicale.

La figure 3 illustre le principe de la méthode de l'invention.

Sur cette figure, les contours du corps du patient 16 sont représentés, en coupe transversale, par une ellipse 30 de centre C. Toutefois, cet exemple n'est pas limitatif et les contours du corps du patient 16 pourront être modélisés et représentés par une forme quelconque. Les poumons et le coeur du patient sont respectivement référencés 32 et 33.

Le but de l'examen est de recueillir une information provenant de l'intérieur de l'ellipse 30.

Pour l'acquisition d'une projection en émission ou en transmission, une tête détectrice 9, 10, dont la surface de détection 25 est symbolisée par une double flèche, est placée à proximité dudit corps 16. Selon l'invention, on acquiert au moins deux projections du corps du patient 16. Une première projection P1 est acquise dans une première position P1 de la surface de détection 25 de la tête détectrice 9 ou 10 par rapport au corps du patient 16, puis une seconde projection P2 est acquise dans une seconde position P2 de ladite surface 25 de cette même tête détectrice 9 ou 10 par rapport audit corps 16. Les deux positions P1, P2 sont angulairement voisines, c'est-à-dire que l'angle α que fait la normale 34 à la surface de détection 25 dans la première position P1 et la normale 35 à ladite surface 25 dans la position P2, est faible, inférieur à 10° environ, en pratique de l'ordre de 3°. Les normales 34, 35 aux surfaces de détection 25 se croisent en un centre C' qui n'est pas obligatoirement le centre C de l'ellipse 30.

On notera que l'angle α peut être égal à 0. Dans ce cas, les normales 34, 35 sont parallèles et il n'existe pas de centre C'.

La première projection P1 est tronquée. En effet, la surface de détection 25, en pratique de l'ordre de 350 x 250 cm, n'est pas suffisante pour permettre une détection de la totalité du corps du patient 16. De ce fait, une partie H1 hachurée, qui ne fait pas partie du champ de détection de la tête 9, 10, n'est pas détectée. Par contre, la seconde projection P2 du corps du patient 16 est acquise dans des conditions telles qu'une portion de la partie H1 est détectée. Une information acquise dans la seconde projection P2 ne la donc pas été dans la première P1.

Selon l'invention, on introduit ou on interpole ladite information dans la première projection P1 en vue de corriger celle-ci dans sa partie tronquée. Réciproquement, dans le cas où la seconde projection P2 est tronquée dans une partie H2, on introduit une information de la première projection dans la seconde projection P2 en vue de corriger cette dernière dans sa partie tronquée. Autrement dit, l'information manquante dans la partie tronquée d'une projection est rajoutée à partir d'une autre projection acquise selon un angle voisin.

On notera que l'introduction d'une information d'une projection dans une autre projection décalée angulairement n'est pas évidente en soi. En effet, si la surface de détection 25 de la tête détectrice 9, 10 avait été suffisamment large pour saisir la totalité du corps du patient 16, elle aurait permis d'acquérir, dans la projection P2, un segment AB de la partie H2. Toutefois, dans l'invention, ce segment AB n'a pas été acquis et on le remplace, dans ladite projection P2, par un segment AC acquis lors de la projection P1. P1 et P2 n'ont pas les mêmes dimensions ni les mêmes orientations dans le corps 16 du patient. Cependant, et notamment pour des projections angulaires voisines des surfaces de détection 25, ces segments AB, AC se confondent ou du moins, tendent vers un segment identique, ce qui permet de corriger les troncatures.

D'autre part, afin de saisir la totalité du corps du patient 16, on déplace les têtes détectrices 9, 10 d'un bord à l'autre du corps du patient 16. On procède donc de sorte que la première projection P1 comprenne l'image d'un bord du corps du patient 16 et que la seconde projection P2 comprenne l'image du bord opposé dudit corps 16. La totalité du corps du patient 16 est alors acquise dans les deux projections P1 et P2 angulairement voisines et l'information d'une projection étant introduite dans une autre et réciproquement, chaque projection du corps P1 ou P2 du patient 16 est acquise virtuellement en totalité. C'est la raison pour laquelle on peut affirmer que le corps du patient 16 est vu dans une zone angulaire virtuelle de 180°. En ce sens, les têtes détectrices 9, 10 possèdent un grand champ de vue virtuel.

En outre, la méthode de l'invention est avantageusement améliorée en imposant une continuité entre les informations introduites dans une partie tronquée d'une projection et celles acquises dans ladite projection.

L'invention va maintenant être décrite relativement à l'exemple d'un examen de tomographie cardiaque avec correction d'atténuation. Dans un tel examen, les têtes détectrices 9, 10 sont avantageusement placées dans une position dite à 90°, dans laquelle les surfaces de détection 25 desdites têtes 9, 10 sont disposées à angle droit l'une de l'autre. Dans ce cas, on définit, pour chaque surface détectrice 25, un bord intérieur 37, c'est-à-dire le bord le plus proche de la surface de détection 25 de la tête voisine, et un bord extérieur 38 opposé au bord intérieur 37. Une telle position est montrée en figure 1. Les têtes détectrices 9, 10 sont alors déplacées en rotation autour du corps du patient 16, entraînées pas à pas à des angles de vue réguliers par l'embase 2 tournante autour de son axe 4. On acquiert ainsi, à chaque angle de vue α(i), une projection en émission par tête détectrice 9, 10 et, dans le cas où on a placé une source radioémettrice gamma dans le boîtier 15 situé face aux têtes détectrices 9, 10, une projection en transmission par tête. L'ensemble des projections permet alors de reconstruire une image du corps du patient 16 corrigée en atténuation.

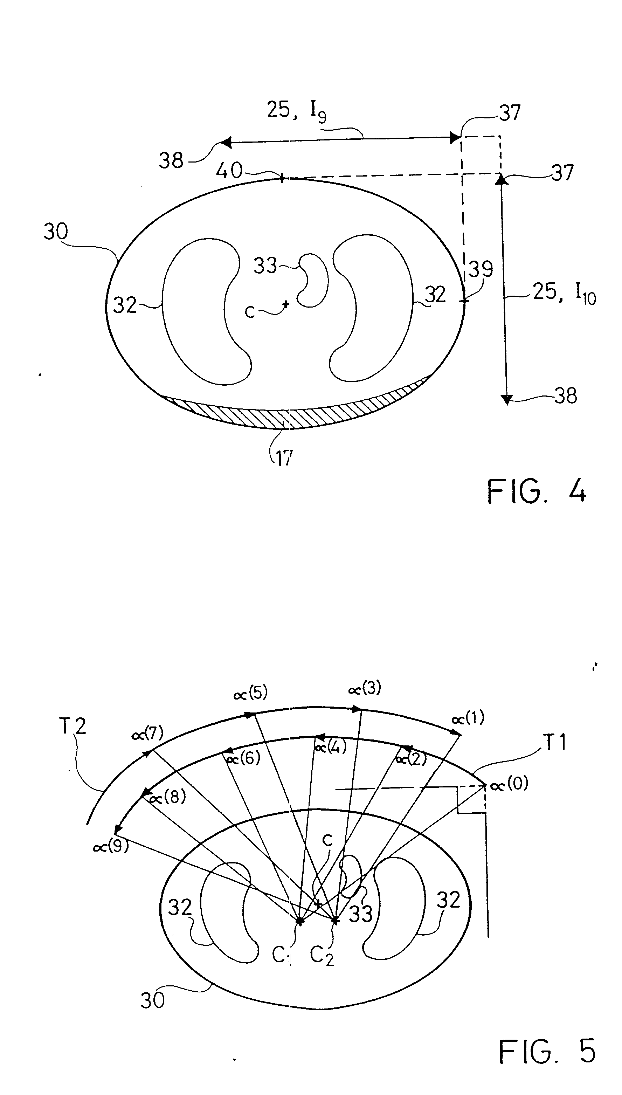

Dans une phase préalable à cet examen illustrée en figure 4, on effectue un positionnement initial des têtes détectrices 9, 10 par rapport au corps du patient 16. A cet effet, on dispose les têtes 9, 10 dans des positions respectives I₉ et I₁₀ qui, moyennant l'hypothèse que le corps du patient 16 est centré sur le lit 17, permettent de déterminer complètement les dimensions du système corps du patient 16 et plateau du lit 17 ainsi que le centre C dudit patient.

Dans un exemple particulièrement avantageux, la tête détectrice 9 est placée dans une position I₉ horizontale et au dessus du corps du patient 16 de manière à ce que le bord intérieur 37 de la surface de détection 25 soit à la verticale d'un point extrême 39 côté coeur 33 du contour 30 du corps du patient 16, et la tête détectrice 10 est placée dans une position I₁₀ verticale (à 90° de la tête 9) et du côté du coeur 33 du patient, de manière à ce que le bord intérieur 37 de sa surface de détection 25 soit à l'horizontale d'un point extrême supérieur 40 du contour 30 du corps du patient 16. Ainsi, les contours du corps du patient 16 disposé sur son lit 17 seront totalement définis, c'est-à-dire modélisés.

Par ailleurs, il est possible d'imposer que le coeur 33 ne doit jamais être tronqué. Cette contrainte peut entraîner une légère troncature du bord de corps du patient 16 opposé audit coeur 33 qui n'aura pas de conséquence majeure sur l'image reconstruite obtenue.

En définitive, la trajectoire intérieure est basée sur la connaissance des dimensions verticale et horizontale du système patient/lit dont la forme est modélisée par une ellipse. Les dimensions de l'ellipse sont obtenues en positionnant manuellement la caméra sur la trajectoire intérieure pour un angle particulier tel qu'un détecteur soit horizontal et au dessus du patient et l'autre soit vertical sur le côté du patient. Dans cette position particulière, le centre de visée peut être défini arbitrairement comme étant, par exemple, le centre de l'ellipse ou encore un organe particulier dont la localisation est définie manuellement. Dès lors, pour chaque angle de vue, la trajectoire est déterminée par la position de la caméra qui assure que les deux droites orthogonales passant alternativement par les bords intérieurs puis les bords extérieurs des champs de détection, soient tangentielles à l'ellipse. Enfin, pour chaque angle de vue, la projection géométrique du centre de visée sur chaque détecteur est translatée selon l'axe des abscisses afin qu'elle coïncide avec le centre de l'image. Accessoirement, dans le cas où la position de l'organe à observer est connue, un contrôle est réalisé pour garantir que l'organe lui-même ne subisse pas de troncature, cette contrainte étant d'ailleurs plus prioritaire que la contrainte de non-troncature du patient.

En fait, la phase préparatoire terminée, il est possible de calculer les positions α(i) pour i variant de 0 à n qu'occuperont chacune des têtes détectrices 9, 10 au cours de l'examen tomographique. Le nombre n de ces positions et le temps des acquisitions est le même que dans le cas d'un examen tomographique s'effectuant sans correction des troncatures. En effet, p des n positions s'inscrivent: selon une première trajectoire T1 des têtes détectrices 9, 10 et n-p autres positions s'inscrivent selon une seconde trajectoire T2. Ces trajectoires T1 et T2, représentées en figure 5, sont avantageusement effectuées successivement. L'une des trajectoires, par exemple la première trajectoire T1, débute à α(0) et se termine α(8). 5 x 4 = 20 projections seront acquises durant cette trajectoire aux positions α(0), α(2), α(4), α(6) et α(8), 5 projections en émission et 5 projections en transmission pour la tête détectrice 9 et de même pour la tête détectrice 10. L'autre trajectoire T2 permet quant à elle d'acquérir 20 projections aux positions α(9), α(7), α(5), α(3) et α(1). Selon l'invention, la trajectoire T1 est dite trajectoire intérieure puisqu'elle est telle que le bord dit intérieur du champ de détection de la tête détectrice suit les contours du corps du patient 16. Par contre, la trajectoire T2 est dite trajectoire extérieure puisqu'elle est telle que le bord du champ de détection opposé au bord intérieur suit les contours du corps du patient 16. Contrairement à la trajectoire intérieure, la trajectoire extérieure ne peut pas se mettre a priori sous une forme analytique.

On notera que, selon l'invention, et afin que les trajectoires T1 et T2 puissent être rigoureusement suivies, la position du corps du patient 16 est modifiée, d'une part, en translation verticale par une montée ou une descente du lit 17 porté par son pied élévateur 19 et, d'autre part, en translation horizontale par le déplacement du bâti 3 le long des rails 36.

Afin de simplifier la figure 5, les projections acquises durant la trajectoire T1 sont représentées centrées sur un centre C1, tandis que celles acquises durant la trajectoire T2 sont représentées centrées sur un centre C2. Cependant, dans le cas général, il n'existe pas de centre unique C1 ou C2 pour une trajectoire T1 ou T2.

Les projections acquises dans les positions α(0), α(2), α(4), α(6) et α(8) sont angulairement voisines des projections acquises dans les positions α(1), α(3), α(5), α(7) et α(9) respectivement.

Si l'une des projections en émission ou en transmission acquise en position α(i) est tronquée, on introduit alors une information de la projection en émission ou en transmission correspondante acquise en position α(i-1) ou α(i+1) dans ladite projection acquise en α(i) afin de la corriger dans sa partie tronquée. Bien entendu, on peut introduire, dans la projection acquise en α(i), des informations acquises à la fois dans les positions α(i-1) et α(i+1).

L'image tomographique reconstruite 28 obtenue ne présentera alors pas d'artefacts dûs aux troncatures.

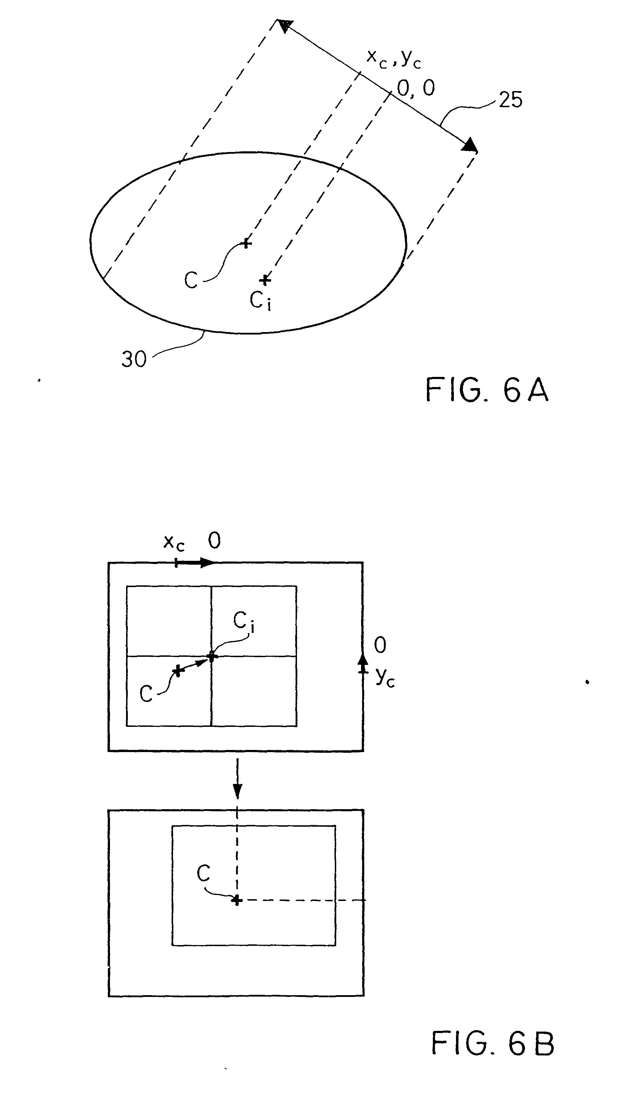

On avait vu, lors de l'explication du principe de l'invention de la figure 3, que le centre de croisée des normales 34, 35 aux surfaces de détection 25 n'était pas obligatoirement le centre C de l'ellipse 30 du corps du patient 16 qui se confond, par hypothèse, avec le centre de l'image reconstruite 28. Or, le centre C choisi doit se projeter au centre de chaque image de projection pour chaque angle de projection. Ceci est réalisé naturellement lors d'une trajectoire circulaire de la ou des têtes détectrices 9, 10 autour du corps du patient 16, mais cela n'est plus vrai pour les trajectoires complexes T1 et T2 pour lesquelles la projection du centre C se décale différemment d'un angle au suivant. Aussi, et pour chaque acquisition représentée en figure 6A correspondant à un angle de vue donné, on décale les images de projection pour faire coïncider la projection du centre C du corps du patient repéré par ses coordonnées x_{C} et y_{C} avec le centre Ci de la projection de coordonnées 0 et 0. Cela peut s'effectuer grâce à une méthode de correction expliquée en figure 6B. Dans cette figure, la projection du centre C, qui apparaît à l'abscisse x_{C} et à l'ordonnée y_{C} de l'image de projection, est décalée d'une quantité -x_{C} et -y_{C}.

## Revendications

1. Méthode d'obtention, en médecine nucléaire, d'une image du corps d'un patient (16), selon laquelle
- on place le corps du patient (16) sur un lit (17) d'une machine de médecine nucléaire (1) comportant au moins une tête détectrice (9 ou 10) munie d'une surface de détection (26) de rayonnements nucléaires (γ) ;
- on acquiert, avec une tête détectrice (9 ou 10), une première image en projection (P1) tronquée du corps du patient (16);
- on acquiert, avec ladite tête détectrice (9 ou 10), une seconde image en projection (P2) du corps du patient (16) et,
- on introduit une information de ladite seconde image (P2) dans ladite première image (P1) en vue de la corriger dans sa partie tronquée (H1);
**caractérisée en ce que**, pour un segment donné appartenant à une partie tronquée de ladite première image en projection (P1), on remplace ce dernier par un segment, obtenu de ladite seconde image en projection (P2), n'ayant pas la même orientation que ledit segment donné, la différence d'orientation correspondant à la différence de la position angulaire (α) de la tête détectrice (9 ou 10) entre l'acquisition de ladite première image et ladite seconde image, les positions angulaires respectives étant voisines dans la mesure où le segment donné et le segment obtenu de ladite seconde image en projection se confondent ou du moins tendent vers un segment identique, de manière à permettre de corriger les troncatures.

2. Méthode selon la revendication 1, **caractérisée en ce que** la seconde image en projection (P2) acquise est tronquée (H2), et en ce que on introduit une information de la première image en projection (P1) dans ladite seconde image (P2) en vue de la corriger dans sa partie tronquée (H2).

3. Méthode selon l'une des revendications 1 ou 2, **caractérisée en ce que** ladite différence de la position angulaire (α) de la tête détectrice (9 ou 10) entre l'acquisition de ladite première image et ladite seconde image est inférieure à 10° environ.

4. Méthode selon l'une des revendications 1, 2 ou 3, **caractérisée en ce que** la première image en projection (P1) acquise comprend l'image d'un bord du corps du patient (16), et en ce que la seconde image en projection (P2) acquise comprend l'image du bord opposé du corps du patient (16).

5. Méthode selon l'une des revendications 1, à 4, **caractérisée en ce que** la totalité du corps du patient (16) est acquise au moyen des deux projections (P1,P2) angulairement voisines de manière à permettre d'obtenir une continuité dans les informations introduites dans une partie tronquée d'une projection et celles acquises dans ladite projection.

6. Méthode selon l'une des revendications précédentes, **caractérisée en ce que** les images en projection sont acquises en tomographie.

7. Méthode selon la revendication 6, **caractérisée en ce que** la tomographie est une tomographie cardiaque avec correction d'atténuation, les têtes détectrices (9, 10) étant disposées dans une position dite à 90°.

8. Méthode selon l'une des revendications 6 ou 7, **caractérisée en ce que**, préalablement à l'examen tomographique, on dispose chaque tête détectrice (9, 10) dans une position (I₉, I₁₀) permettant de déterminer les dimensions du corps du patient ainsi que son centre C.

9. Méthode selon la revendication 8, **caractérisée en ce que** l'on impose que le coeur (33) ne doit jamais être tronqué.

10. Méthode selon l'une des revendications précédentes, **caractérisée en ce que** l'image reconstruite (28) est une image tomographique obtenue par l'acquisition d'images en projection du corps du patient prises sous différentes angles α(i).

11. Méthode selon l'une des revendications 6 à 10, **caractérisée en ce que** p des n images en projection sont acquises selon une première trajectoire (T1) de la (des) tête(s) détectrice(s) (9, 10) et n-p autres images sont acquises selon une seconde trajectoire (T2) de ladite (lesdites) tête(s) (9, 10).

12. Méthode selon la revendication 11, **caractérisée en ce que** l'une des trajectoires, par exemple la première trajectoire (T1), dite trajectoire intérieure, est telle qu'un bord dit intérieur du champ de détection de la tête détectrice suit les bords du corps du patient (16).

13. Méthode selon l'une des revendications 11 ou 12, **caractérisée en ce que** la seconde trajectoire (T2), dite trajectoire extérieure, est telle qu'un bord du champ de détection, opposé au bord dit intérieur, suit les bords du corps du patient (16).

14. Méthode selon l'une des revendications précédentes, **caractérisée en ce que** les images en projection acquises le sont en émission ou en transmission.

## Patentansprüche

1. Nuklearmedizinisches Verfahren zur Gewinnung eines Bildes des Körpers eines Patienten (16), nach welchem Verfahren:
- der Körper des Patienten (16) auf ein Bett (17) einer nuklearmedizinischen Anlage (1) gelegt wird, die mindestens einen Detektorkopf (9 oder 10) mit einer Nachweisoberfläche (26) für Nuklearstrahlung (γ) besitzt;
- mit einem Detektorkopf (9 oder 10) ein erstes, gekürztes Projektionsbild (P1) des Körpers des Patienten (16) gewonnen wird;
- mit dem genannten Detektorkopf (9 oder 10) ein zweites Projektionsbild (P2) des Körpers des Patienten (16) gewonnen wird;
- eine Information des genannten zweiten Bildes (P2) in das genannte erste Bild (P1) eingeführt wird, um dieses in seinem gekürzten Teil (H1) zu korrigieren;
**dadurch gekennzeichnet**, dass bei einem zu einem gegebenen gekürzten Teil des genannten ersten Projektionsbildes (P1) gehörigen Segment das letztere durch ein Segment ersetzt wird, das aus dem genannten zweiten Projektionsbild (P2) herrührt, das nicht dieselbe Orientierung aufweist wie das genannte gegebene Segment, wobei der Orientierungsunterschied der Differenz der Winkelstellung (α) des Detektorkopfs (9 oder 10) zwischen der Gewinnung des genannten ersten Bildes und des genannten zweiten Bildes entspricht, welche jeweiligen Winkelstellungen insoweit ähnlich sind, als das gegebene Segment und das in dem zweiten Projektionsbild gewonnene Segment zusammenfallen oder zumindest dazu neigen, ein einheitliches Segment zu bilden, so dass eine Korrektur der abgeschnittenen Bildteile möglich wird.

2. Verfahren nach Anspruch 1, **dadurch gekennzeichnet**, dass das gewonnene zweite Projektionsbild (P2) gekürzt (H2) ist, und dass eine Information aus dem ersten Projektionsbild (P1) in das genannte zweite Bild (P2) eingeführt wird, um es in seinem gekürzten Teil (H2) zu korrigieren.

3. Verfahren nach einem der Ansprüche 1 oder 2, **dadurch gekennzeichnet**, dass der genannte Unterschied der Winkelstellung (α) des Detektorkopfs (9 oder 10) zwischen der Gewinnung des genannten ersten Bildes und des genannten zweiten Bildes weniger als etwa 10° beträgt.

4. Verfahren nach einem der Ansprüche 1, 2 oder 3, **dadurch gekennzeichnet**, dass das erste gewonnene Projektionsbild (P1) das Bild eines Randes des Körpers des Patienten (16) enthält, und dass das gewonnene zweite Projektionsbild (P2) das Bild des gegenüberliegenden Randes des Körpers des Patienten (16) enthält.

5. Verfahren nach einem der Ansprüche 1 bis 4, **dadurch gekennzeichnet**, dass der vollständige Körper des Patienten (16) mit Hilfe der beiden in ihrer Winkellage benachbarten Projektionsbilder (P1, P2) in der Weise aufgenommen wird, dass es möglich ist, einen stetigen Übergang von den in einen gekürzten Teil einer Projektion eingebrachten Informationen zu den Informationen zu erhalten, die in der genannten Projektion gewonnen wurden.

6. Verfahren nach einem der vorhergehenden Ansprüche, **dadurch gekennzeichnet**, dass die Projektionsbilder in einem Tomographieverfahren gewonnen werden.

7. Verfahren nach Anspruch 6, **dadurch gekennzeichnet**, dass die Tomographie eine Herztomographie mit Dämpfungskorrektur ist, wobei die Detektorköpfe (9, 10) in sogenannter 90°-Position angeordnet sind.

8. Verfahren nach einem der Ansprüche 6 oder 7, **dadurch gekennzeichnet**, dass jeder Detektorkopf (9, 10) vor der tomographischen Untersuchung in eine Position (I₉, I₁₀) gebracht wird, die es ermöglicht, die Abmessungen des Körpers des Patienten sowie sein Zentrum C zu bestimmen.

9. Verfahren nach Anspruch 8, **dadurch gekennzeichnet**, dass festgelegt wird, dass das Herz (33) in keinem Fall angeschnitten wird.

10. Verfahren nach einem der vorhergehenden Ansprüche, **dadurch gekennzeichnet**, dass das wiederaufgebaute Bild (28) ein tomographisches Bild ist, das durch die Erfassung von Projektionsbildern des unter verschiedenen Winkeln α(i) aufgenommenen Körpers des Patienten gewonnen wurde.

11. Verfahren nach einem der Ansprüche 6 bis 10, **dadurch gekennzeichnet**, dass p der n Projektionsbilder längs einer ersten Bahn (T1) des (der) Detektorkopfs (Detektorköpfe) (9, 10) und n-p weitere Bilder längs einer zweiten Bahn (T2) des (der) genannten Detektorkopfs (Detektorköpfe) (9, 10) aufgenommen werden.

12. Verfahren nach Anspruch 11, **dadurch gekennzeichnet**, dass die eine der Bahnlinien, beispielsweise die als innere Bahnlinie bezeichnete Bahnlinie (T1), so verläuft, dass ein als Innenrand bezeichneter Rand des Erfassungsfeldes des Detektorkopfs den Rändern des Körpers des Patienten (16) folgt.

13. Verfahren nach einem der Ansprüche 11 oder 12, **dadurch gekennzeichnet**, dass die zweite, als äußere Bahnlinie bezeichnete Bahnlinie (T2) so verläuft, dass ein dem als Innenrand bezeichneten Rand gegenüberliegender Rand des Erfassungsfeldes den Rändern des Körpers des Patienten (16) folgt.

14. Verfahren nach einem der vorhergehenden Ansprüche, **dadurch gekennzeichnet**, dass die gewonnenen Projektionsbilder Emissions- oder Transmissionsbilder sind.

## Claims

1. Method in nuclear medicine for obtaining an image of the body of a patient (16) according to which:
- the body of the patient (16) is placed on a bed (17) of a nuclear medicine machine (1) comprising at least one detecting head (9 or 10) provided with a detection surface (26) for nuclear radiation (γ);
- a first truncated projection image (P1) of the body of the patient (16) is acquired with one detecting head (9 or 10);
- a second projection image (P2) of the body of the patient (16) is acquired with said detecting head (9 or 10), and
- information from said second image (P2) is introduced into said first image (P1) in order to correct it in its truncated portion (H1);
**characterised in that**, for a given segment belonging to a truncated portion of said first projection image (P1), the latter is replaced by a segment obtained from said second projection image (P2) not having the same orientation as said given segment, the difference in orientation corresponding to the difference in the angular position (α) of the detecting head (9 or 10) between acquisition of said first image and said second image, the respective angular positions being close to the extent that the given segment and the segment obtained from said second projection image merge into one another or at least tend towards an identical segment so as to permit correction for truncation.

2. Method according to claim 1, **characterised in that** the second projection image (P2) acquired is truncated (H2), and in that information from the first projection image (P1) is introduced into said second image (P2) in order to correct it in its truncated portion (H2).

3. Method according to one of claims 1 or 2,
**characterised in that** said difference in the angular position (α) of the detecting head (9 or 10) between acquisition of said first image and said second image is approximately less than 10°.

4. Method according to one of claims 1, 2 or 3,
**characterised in that** the first projection image (P1) acquired comprises the image from one side of the body of the patient (16), and in that the second projection image (P2) acquired comprises the image from the opposite side of the body of the patient (16).

5. Method according to one of claims 1 to 4,
**characterised in that** the totality of the body of the patient (16) is acquired by means of the two angularly close projections (P1, P2) so as to make it possible to obtain continuity in the information introduced into a truncated portion of a projection and that acquired in said projection.

6. Method according to one of the preceding claims,
**characterised in that** the projection images are acquired by tomography.

7. Method according to claim 6, **characterised in that** the tomography is cardiac tomography corrected for attenuation, the detecting heads (9, 10) being disposed in a so-called position at 90°.

8. Method according one of claims 6 or 7, **characterised in that**, prior to the tomographic examination, each detecting head (9, 10) is disposed in a position (I₉, I₁₀) making it possible to determine the dimensions of the body of the patient and its centre C.

9. Method according to claim 8, **characterised in that** it is laid down that the heart (33) must never be truncated.

10. Method according to one of the preceding claims,
**characterised in that** the reconstructed image (28) is a tomographic image obtained through acquisition of projection images of the body of the patient taken at different angles α(i).

11. Method according to one of claims 6 to 10,
**characterised in that** p of the n projection images are acquired with a first trajectory (T1) of the detecting head(s) (9, 10) and n-p other images are acquired with a second trajectory (T2) of said head(s) (9, 10).

12. Method according to claim 11, **characterised in that** one of the trajectories, for example the first trajectory (T1), the so-called inner trajectory, is such that a so-called inner side of the detection field of the detecting head follows the sides of the body of the patient (16).

13. Method according to one of claims 11 or 12,
**characterised in that** the second trajectory (T2), the so-called outer trajectory, is such that one side of the detection field opposite the so-called inner side, follows the sides of the body of the patient (16).

14. Method according to one of the preceding claims,
**characterised in that** the projection images acquired are acquired by emission or transmission.
